Europäisches Patentamt

European Patent Office

Office européen des brevets

(19)

(11) **EP 1 473 273 A1**

(12) ## EUROPEAN PATENT APPLICATION

(43) Date of publication:
**03.11.2004 Bulletin 2004/45**

(51) Int Cl.7: **C01B 25/32**

(21) Application number: **03026168.9**

(22) Date of filing: **17.11.2003**

| | |
|---|---|
| (84) Designated Contracting States:<br>**AT BE BG CH CY CZ DE DK EE ES FI FR GB GR<br>HU IE IT LI LU MC NL PT RO SE SI SK TR**<br>Designated Extension States:<br>**AL LT LV MK** | (71) Applicant: **Biomet Deutschland GmbH<br>14167 Berlin (DE)** |
| (30) Priority: **02.05.2003 EP 03010026** | (72) Inventor: **Tas, Ahmet Cuneyt, Dr.<br>Batikent 06370 Ankara (TR)** |

(54) **Method of preparing alpha-and beta-tricalcium phosphate powders**

(57)    The invention describes a wet-chemical process for the preparation of alpha- and beta-tricalcium phosphate (TCP) powders. The procedure simply comprises immediate addition of di-ammonium hydrogen phosphate to an aqueous solution of calcium nitrate tetrahydrate, or vice versa. Calcination of the recovered precursor powders at 800°C in air produces beta-TCP, whereas the calcination of the same at 1200°C, followed by rapid cooling to room temperature yields submicron-particulated alpha-TCP powders. These powders can be the raw materials for bioceramics, such as artificial bones, artificial joints, artificial tooth roots, and calcium phosphate-based self-setting, self-hardening cements.

EP 1 473 273 A1

**Description**

The invention relates to a method of preparing $\alpha$- and $\beta$-tricalcium phosphate (TCP) powders of submicron particle size. These powders can be the raw materials for bioceramics, such as artificial bones, artificial joints, artificial tooth roots, and calcium phosphate-based self-setting, self-hardening cements.

Alpha-tricalcium phosphate ($\alpha$-TCP) is the high-temperature and beta-tricalcium phosphate ($\beta$-TCP) is the low-temperature polymorph of this important bioceramic material. The polymorphic transformation of $\beta$-TCP (upon heating) into $\alpha$-TCP is observed at the temperature of around 1180°C. $\alpha$-TCP formed at temperatures higher than 1180°C can not be preserved upon slow cooling to room temperature, and it can only be obtained at RT by rapid cooling or quenching.

$\beta$-TCP has relatively higher solubility (or resorbability) in living bodies, as compared to $\alpha$-TCP. On the other hand, $\alpha$-TCP powders have the unique ability of self-hardening (compressive strength of >10 MPa) when mixed with a proper amount of a suitable setting/hardening solution, and this form of TCP is heavily preferred and used in many of the commercially available calcium phosphate cement formulations. Both forms of TCP bioceramics are shown to be bioactive and allow new bone formation around them (without displaying formation of in vivo fibrous tissue formation) by cellular remodelling. For fast and complete resorption (6 to 8 months following implantation) of the implant materials, the material of choice would be $\beta$-TCP.

For the further preparing of various forms of porous or non-porous TCP, one needs to start with fine powders of these phases, i.e., which have physical (in terms of particle size and shape distribution) and chemical (in terms of the consistency of Ca/P molar ratio and elemental distribution/purity) homogeneity along the strict entirety of the powder body.

As means of producing both polymorphic forms of TCP, dry methods (i.e., "solid-state reactive firing" (SSRF) of more than one components, whereas each component may respectively serve as the calcium- and the phosphate-source; such as $CaCO_3 + CaHPO_4$, or $CaCO_3+(NH_4)H_2PO_4$, etc.) are available. The major steps in the dry methods of TCP synthesis can be listed as follows; 1) the intimate, physical "mixing" of two (or sometimes more) components to achieve a homogenous reactant body prior to the start of heating cycles, 2) "compaction" of the starting materials (by using pressing or granulation processes) into dense pellets, tablets or granules to decrease the diffusion distances between the individual tiny particles of the reactants, 3) full conversion of the reactant two-phase mixture at a sufficiently high-temperature (1300° to 1400°C) of "firing or sintering" into single-phase TCP, 4) "crushing and grinding" of the sintered product to have an average particle size in the vicinity of 1 $\mu$m. All of these steps of mixing, compaction, sintering and grinding are expensive, laborintensive, time-consuming, and tedious. Most of the time, repeated sintering+grinding steps (i.e., steps 3 and 4) need to be incorporated into the process flowchart to achieve the desired phase purity.

A few of wet methods of TCP synthesis are also known. The most preferred route of TCP precursor powder synthesis (see U.S. Patent 5,011,495) has been the mixing of calcium hydroxide, $Ca(OH)_2$, or $CaCO_3$, together with phosphoric acid ($H_3PO_4$) to form a slurry, followed by aging of that slurry (which is required for the neutralization reaction to go to completion) for a relatively long time at temperatures between 60° to 90°C (typically requiring the use of an autoclave). The precursor powders formed by this way were later calcined at temperatures higher than 800°C to convert them into single-phase TCP. The major drawback of this process is the occlusion of still unreacted $Ca(OH)_2$ particles in the cores of the formed TCP particles, which eventually leads to a heterogeneity in terms of the atomic Ca/P ratio of the final product powders.

As an other procedure of wet synthesis of TCP, sol-gel synthesis can be mentioned (see J. Livage, P. Barboux, M. T. Vandenborre, C. Schmutz, and F. Taulelle, "Sol-Gel Synthesis of Phosphates," J. Non-Cryst. Solids, 147/148, pp. 18-23, 1992). In this method, typically, $CaCl_2$ (or $Ca(NO_3)_2 \cdot 4H_2O$) and triethylphosphate ($C_6H_{15}O_4P$) are reacted to form a colloidal sol, which was then forced to go through the steps of hydrolysis, polycondensation and gelation, followed by calcination of the obtained gel at high temperatures. The major disadvantages of this procedure are (i) the high costs associated with the use of triethylphosphate, and (ii) the necessity of using a carefully designed chemical reactor (which is not yet shown to be practical on an industrial scale) for the homogeneous sol formation.

An object of the present invention is to provide a simple method for preparing alpha- and beta-TCP powders of sub-micron particle size, which avoids the above-mentioned disadvantages from the prior art. Upon further study of the specification and appended claims, further objects and advantages of this invention will become apparent to those skilled in the art.

These objects are achieved by a simple method of preparing beta- and alpha-TCP powders of sub-micron particle size characterized in that the method comprising the steps of:

a) Adding an aqueous solution of $Ca(NO_3)_2$ x $4H_2O$ to an aqueous solution of $(NH_4)_2HPO_4$ under stirring
b) slowly adding of concentrated $NH_4OH$ solution to ensure the formation of apatitic tricalcium phosphate ($Ca_9(HPO_4)(PO_4)_5OH$) under stirring

c) filtering, washing and drying the precipitates
d) calcining the powder at 800 °C and 1200 °C (for alpha-TCP) respectively followed by cooling to obtain single-phase beta- (and alpha-)TCP powders.

By the nature of aqueous chemistry of calcium phosphate phase system (i.e., $CaO$-$P_2O_5$-$H_2O$ ternary system), it is theoretically not possible to form TCP, $Ca_3(PO_4)_2$, powders in a single-step aqueous, chemical precipitation process. Therefore, the best thing to do would remain as the ability to form the sub-micron precipitate of $Ca_9(HPO_4)$ $(PO_4)_5OH$, which is also named as "apatitic tricalcium phosphate," having a Ca/P ratio of 1.50, and then convert it to TCP by calcination (as a loose powder, i.e., there is no need for compaction of the powders) at a relatively low temperature.

Low temperature calcination, then, would not destroy the chemical composition of the precipitates, and this calcination would only cause the evaporation of 1 molecular unit of $H_2O$ from 1 formula unit of the apatitic tricalcium phosphate, according to the below, hypothetical reaction:

$$Ca_9(HPO_4)(PO_4)_5OH = Ca_9(PO_4)_6 \cdot H_2O \rightarrow 3\, Ca_3(PO_4)_2 + H_2O.$$

This reaction involves a slight change in the crystal structure of the initial precipitates, therefore, sufficient time must be allowed at the temperature to push the reaction to completion.

The advantage of the present invention is to provide simple methods for inexpensive commercial preparing of chemically homogeneous, single-phase powders of

(i) apatitic tricalcium phosphate ($Ca_9(HPO_4)(PO_4)_5OH$),
(ii) β-TCP, and
(iii) α-TCP.

The first and second of these fine powders are suitable for the production of fast resorbing *(in vivo)*, porous or non-porous, bioceramic implant materials of different forms to help in the processes of bone defect healing and bone remodelling. The last of these powders (α-TCP) is to be used in the preparation of calcium phosphate self-setting/self-hardening cements. To be specific, the present invention relates to a wet-chemical method for the production of the above by starting with an aqueous solution mixture of calcium nitrate tetrahydrate and di-ammonium hydrogen phosphate. Calcination temperature selected and the cooling rate employed during the further processing of the recovered precipitates simply govern the polymorphic form (α or β) of the TCP powder to be obtained. Powders obtained (according to the working examples given below) of either alpha-or beta-TCP form do not require high-energy crushing/grinding, and even after calcination they already consist of fluffy agglomerates of submicron particulates. Submicron particles mean particles which have a size of 0.3 to 0.4 microns.

Detailed description of the invention

In the method of the present invention, first an aqueous solution (most preferably in the concentration range of 0.20 to 0.25 M) of di-ammonium hydrogen phosphate (($NH_4)_2HPO_4$) is prepared by simply dissolving the inorganic salt powder in distilled water. A clear solution is formed. The temperature of synthesis is not so critical on the physical and chemical characteristics of the powders to be obtained, and it can preferably be adjusted between room temperature (18° to 22°C) and the physiological body temperature of 37°C.

An appropriate quantity (an amount to make the Ca/P molar ratio in the solution to be exactly equal to 1.50) of calcium nitrate tetrahydrate ($Ca(NO_3)_2 \cdot 4H_2O$) powder is then added at once into the above solution. Upon addition of calcium nitrate, the solution immediately becomes opaque, and precipitates form. The chemical nature of the formed precipitates at this stage is governed by the solution pH value. A certain amount of concentrated (preferred is 20 to 30 vol%, most preferred is 25 vol%) ammonia water ($NH_4OH$) must be added at once to the reaction mixture to ensure the formation of apatitic tricalcium phosphate ($Ca_9(HPO_4)(PO_4)_5OH$) with continuous stirring for a certain time, following the addition of calcium nitrate powder. If this addition of ammonia water had not been made, the obtained precipitates would be contaminated with phases like $Ca_2P_2O_7$ and $CaHPO_4 \cdot 2H_2O$. The solution was stirred for 120 to 140 minutes, prior to decanting the mother liquor, and filtration of the precipitates. Recovered precipitates were then dried at 60°C, followed by calcination in an air atmosphere to form either beta- or alpha-TCP powders.

The invention is described in detail below in terms of the following working examples.

In the foregoing and in the following examples, all temperatures are set forth uncorrected in degrees Celsius; and, unless otherwise indicated, all parts and percentages are by weight.

**Example 1**

Production of $Ca_9(HPO_4)(PO_4)_5OH$ ("apatitic tricalcium phosphate") precipitates:

257.65 g of $(NH_4)_2HPO_4$ powder was dissolved in 8250 mL of distilled water in a 10 liters-capacity glass container, followed by heating it to about 37°C, under continuous stirring, on a hot-plate. 691.10 g of $Ca(NO_3)_2 \cdot 4H_2O$ powder was then added to the above solution. 160 mL of 25 vol.% $NH_4OH$ solution was poured into the opaque solution within minutes following the addition of calcium nitrate. Solution was mixed for 120 to 140 min at constant temperature. Precipitates were immediately separated from the container by filtration with filter paper and dried at 60°C for 18 to 24 hours.

**Example 2**

Production of β-TCP (β-$Ca_3(PO_4)_2$) powders:

Fine powders produced in Example 1 were placed (spread as loose powders) into aluminum oxide trays, and heated to 800°C (with a heating rate of 5 to 6°C/min) in a electrically-heated chamber furnace and soaked at 800°C for 12 hours. Samples were cooled to room temperature within the said furnace with a cooling rate of 3°C/min. Quite fluffy and submicron powders obtained were single-phase β-TCP (i.e., Whitlockite).

**Example 3**

Production of α-TCP (α-$Ca_3(PO_4)_2$) powders:

Fine powders produced in Example 1 were placed (spread as loose powders) into aluminum oxide trays, and heated to 1200°C (with a heating rate of 5 to 6°C/min) in an electrically-heated chamber furnace and soaked at 1200°C for 3 to 4 hours. Samples were then quenched to 1000°C in 10 minutes within the said furnace (by slightly opening the door of the furnace), followed by cooling to 500°C in no more than 1 h. Powders obtained were single-phase α-TCP.

**Claims**

1. A method of preparing beta-tricalcium phosphate (beta-TCP) powder of sub-micron particle size **characterized in that** the method comprising the steps of:

   a) adding an aqueous solution of $Ca(NO_3)_2$ x $4H_2O$ to an aqueous solution of $(NH_4)_2HPO_4$ under stirring
   b) slowly adding of concentrated $NH_4OH$ solution to ensure the formation of apatitic tricalcium phosphate ($Ca_9(HPO_4)(PO_4)_5OH$) under stirring
   c) filtering, washing and drying the precipitates
   d) calcining the powder at 800 °C followed by cooling to obtain single-phase beta-TCP powders.

2. A method of preparing alpha-TCP powder of sub-micron particle size **characterized in that** the method comprising the steps of:

   a) adding an aqueous solution of $Ca(NO_3)_2$ x $4H_2O$ to an aqueous solution of $(NH_4)_2HPO_4$ under stirring
   b) slowly adding of concentrated $NH_4OH$ solution to ensure the formation of apatitic tricalcium phosphate ($Ca_9(HPO_4)(PO_4)_5OH$) under stirring
   c) filtering, washing and drying the precipitates
   d) calcining the powder at 1200 °C followed by cooling to obtain single-phase alpha-TCP powders

3. The method according to either of the preceding claims **characterized in that** according to step a) the Ca/P ratio in this solution is 1.50.

4. The method according to any of claims 1 to 3 **characterized in that** according to step b) the concentrated $NH_4OH$ solution is 20 to 30 vol. %, preferably 25 vol.%.

5. The method according to any of claims 1 to 4 **characterized in that** according to step a) the aqueous solution of $(NH_4)_2HPO_4$ has a concentration of 0.20 to 0.25 M.

**European Patent Office**

# EUROPEAN SEARCH REPORT

Application Number

EP 03 02 6168

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | SALAHI E ET AL: "Synthesis and thermal behaviour of beta tricalcium phosphate precipitated from aqueous solutions" BR. CERAM. TRANS. (UK), BRITISH CERAMIC TRANSACTIONS, INST. MATER, UK, vol. 102, no. 2, April 2003 (2003-04), pages 79-82, XP008033086 ISSN: 0967-9782 * the whole document * | 1-5 | C01B25/32 |
| A | JARCHO M ET AL: "Synthesis and fabrication of beta -tricalcium phosphate (whitlockite) ceramics for potential prosthetic applications" JOURNAL OF MATERIALS SCIENCE, vol. 14, no. 1, January 1979 (1979-01), pages 142-150, XP008033115 ISSN: 0022-2461 | | |

TECHNICAL FIELDS SEARCHED (Int.Cl.7)

C01B

The present search report has been drawn up for all claims

| Place of search | Date of completion of the search | Examiner |
|---|---|---|
| The Hague | 25 August 2004 | Rigondaud, B |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)